# EUROPEAN PATENT SPECIFICATION

(11) **EP 3 263 020 B1**
(45) Date of publication and mention of the grant of the patent: **29.12.2021**
(21) Application number: 15874605.7
(22) Date of filing: 26.01.2015
(51) Int. Cl.: A61B 5/02, A61B 5/00, A61B 5/021

(54) **OPTICAL FIBER TYPE CONTINUOUS BLOOD PRESSURE DETECTION SENSOR AND WEARABLE DEVICE THEREOF**
FASEROPTISCHER KONTINUIERLICHER BLUTDRUCKDETEKTIONSSENSOR UND AM KÖRPER TRAGBARE VORRICHTUNG DAFÜR
CAPTEUR DE DÉTECTION CONTINUE DE PRESSION ARTÉRIELLE DE TYPE À FIBRES OPTIQUES ET DISPOSITIF PORTABLE ASSOCIÉ

(30) Priority: 31.12.2014 CN 201410854008
(43) Date of publication of application: 03.01.2018
(73) Proprietor: Huijia Health Life Technology Co., Ltd., Hsinchu County, Taiwan 302 (TW)
(72) Inventor: YANG, Shuchen, Hsinchu County, Taiwan 302 (TW)
(74) Representative: Ehrner & Delmar Patentbyrå AB
(86) International application number: PCT/CN2015/071559
(87) International publication number: WO 2016/106912

(56) References cited:
- WO-A1-2011/016778
- WO-A1-2013/051602
- CN-A- 102 573 615
- CN-U- 204 468 056
- JP-A- 2008 284 001
- JP-A- 2012 065 911
- US-A- 5 134 281
- US-A1- 2003 095 263

## Description

### Cross Reference to Related Application

This application is an International Application No. PCT/CN2015/071559 for entry into European regional phase, with an International filing date of January 26, 2015.

### TECHNICAL FIELD

The present application relates to the technical field of blood pressure detection, and particularly to an optical fiber continuous detecting blood pressure sensor and its wearing apparatus.

### BACKGROUND

Nowadays, normally a non-invasive detecting method is provided for detecting blood pressure.

Non-invasive detecting mainly includes the stethoscopy method (Korotkoff-Sound Method) and the succussion method (Oscillography Method). The principle of the stethoscopy method is to collect Korotkoff sound. The whole apparatus includes an inflatable and deflatable cuff, a mercury manometer and a stethoscope. The principle of succussion is to utilize the oscillographic principle of pulse contour to determine the SBP (Systolic Blood Pressure) and the DBP (Diastolic Blood Pressure), which is widely used in most domestic and overseas non-invasive automated sphygmomanometers nowadays.

However, these both the two methods all need to inflate and deflate the cuff. The disadvantages are that: it is difficult to carry the apparatuses because each of them includes the cuff, the pump and the valve or the like and thus has a large volume. If the cuff is used frequently, the tissue and blood vessel beneath the cuff may be damaged because of being frequently pressed; because a certain time for inflating and deflating the cuff is needed, the continuous detection for blood pressure cannot be realized.

Moreover, the recent study result shows that non-cuff non-invasive blood pressure detecting detection is possible. It is a method that utilizes the transmission speed of a pulse wave to detect a blood pressure. The transmission speed of the pulse wave refers to the speed of the pulse wave transmits along the artery. Many study results show that: the pulse wave transmission speed is related to a blood pressure. A common way to detect the pulse wave transmission speed is to detect the pulse wave transmission time which is the time that needed for the pulse wave to transmit from the heart to radial artery. The pulse transmission time could be determined by utilizing a reference point on an electrocardiosignal and another reference point on a pulse wave detected on a peripheral arterial at during the same cardiac cycle. Pulse The pulse wave could be detected through an optical method. It is called as photoplethysmography. Photoplethysmography comprises lighting the light on the body tissue and detecting the reflected light, transmission light or scattered light on the body tissue. The light signal received by the photoelectric detector presents the variation of blood flow volume of the tissue.

However, the detecting device utilize pulse transmission time or speed to detect blood pressure may have the following disadvantages: detecting the pulse wave transmission time needs an ECG (electrocardiograph) detecting device and a pulse wave detecting device, which means many detecting devices are needed; in addition, in order to detect the needed time of the pulse wave transmitted from the heart to the a reference point on radial artery precisely during detection, a subject cannot move freely. If the subject moves, the locating of the reference would be imprecise, then it may need to relocate the reference point. Hence, the present pulse wave detecting apparatus is not suitable for longtime continuous detecting and it only could be used in a clinic.

As disclosed on a patent with authorized announcement No. CN101288587, a strap human blood pressure non-invasive continuous detecting apparatus utilizes a sphere-shape pulse wave sensor probe to detect the pulse wave of a human wrist radial artery ensures a good contact between the probe and the human wrist radial artery by a compressing spring, and through the positioning of the slot and the olecroanon, guarantees that the repositioning would be precise and the wrist movement would be avoided during the continuous detecting process. Furthermore, a prior art document D1 (Pub. No.: US5134281A) discloses an optical sensor comprising an optical fiber having a core covered by a cladding, the cladding having an index of refraction different from that of the core, apparatus for retaining the fiber in a sinuously looped disposition, apparatus for fixing the fiber to an object to be sensed whereby movement to be sensed results in one or both of accordion expansion and contraction of loops of the fiber and micro-bending of the fiber, apparatus for applying a first optical signal into one end of the fiber, apparatus for detecting a resulting optical signal from the other end of the fiber, and apparatus for comparing the first and resulting optical signals to obtain an indication of the movement.

However, there is also such a problem: the area that contacts with the probe is the area where pulse wave sensor detects the pulse wave of the human radial artery, which is a point to point area detection. Therefore, when the strap human blood pressure non-invasive continuous detecting apparatus is used, the probe of the pulse wave sensor has to align with the human wrist radial artery and the slot has to align with the olecroanon. During the detecting process, if the strap moves inevitably due to the movement of the subject's wrist, the positioning of the probe and the human wrist radial artery will be dislocated. Therefore, it will cause the positioning imprecision of the human wrist radial artery relative to the strap human blood pressure non-invasive detecting apparatus, and the 24 hours non-invasive continuous detection for human blood pressure cannot be realized.

### SUMMARY

A purpose of the application is providing an optical fiber continuous detecting blood pressure sensor. It aims to solve the problems in existing blood pressure detecting methods that there are complicated structures, it is difficult to carry, a subject's skin may be damaged, a subject cannot move freely, the positioning is imprecise, and it is unable to perform a continuous detection for a longtime.

In order to solve the above technical issues, a technical solution of an optical fiber continuous detecting blood pressure sensor provided by the application comprises a sensing band which is flexible and can be curled into a ring, wherein, the sensing band includes an inner layer which abuts to the detected surface and an outer layer; an accommodation space is formed between the inner layer and the outer layer; an optical fiber is provided in the accommodation space and extends along the sensing band so as to form a sensing area to sense a pulse wave; a signal processor is provided on one end of the sensing band and used to convert a luminous decay signal generated by decaying a light signal through the optical fiber into a charge unit so as to calculate the pulse wave; the signal processor is communicated with the optical fiber, and an outer surface of the sensing band is covered by an elastic layer.

According to the invention, a corrugated first concave-convex structure is formed on an outer surface of the inner layer.

Further, the shape of the first convex-concave structure is selected from a triangular corrugated shape, a circular corrugated shape or, a quadrate corrugated shape, a trapezoidal corrugated shape, and any combination of at least two of these shapes.

Further, the optical fiber includes an optical fiber row which has several U-shape concatenated cablings extending along the longitudinal direction or width direction of the sensing band.

Further, the optical fiber includes an optical fiber row which has several S-shape concatenated cablings extending along the longitudinal direction or width direction of the sensing band.

Further, the optical fiber includes an optical fiber row which has several O-shape concatenated cablings extending along the longitudinal direction or width direction of the sensing band.

Further, the signal processor includes an optical detecting module used to receive the light decay signal of the optical fiber, a signal calculating and processing module used to convert the light decay signal of the optical fiber into charge unit so as to calculate the pulse wave, a blood pressure calibrating module used to process, analyze and calculate the pulse wave in order to obtain the blood pressure value, a memory module used to store the blood pressure value, and a displaying module used to display the blood pressure value.

Further, at least two optical fiber rows are superposed vertically, and bending portions of the cablings of the two vertically superposed and adjacent optical fiber rows are staggered.

Further, at least two optical fiber rows are superposed vertically, and bending portions of the cablings of the two vertically superposed and adjacent optical fiber rows are staggered transversely and longitudinally.

The application provides an optical fiber continuous blood pressure sensor, which has the following advantages compared with the prior art:

The sensing band is used in the above said optical fiber continuous detecting blood pressure sensor. The sensing band has an accommodation space and the accommodation space is provided therein with an optical fiber. The sensing band is flexible and can be curled into a ring, therefore, an optical fiber is arranged to extend along the sensing band and forms a ring-shape sensing area. The sensing band could be worn on the human arm or wrist, especially worn on the area of human wrist radial artery. When the sensing band on human wrist rotates or dislocates because of the movement of a subject, the sensing area can sense the pulse wave of the human wrist radial artery, which means that special positioning is not needed for wearing the sensing band.

Moreover, the sensing band is covered by an elastic layer which can be contracted elastically at a certain distance extent. In this way, the sensing band can be worn on different human wrist. The elastic layer has certain elasticity, which helps the sensing band remain a certain elastic contact stress on the wrist. Therefore, the sensing band is worn on the wrist securely. During detecting the blood pressure, even the subject moves, the dislocation between the sensing band and the human wrist radial artery is not prone to occur easily and there is no need to reposition the sensing band, which thus a 24 hours non-invasive continuous detecting for a human blood pressure can be realized, which is stable and precise.

Therefore, compared with existing non-invasive detecting methods, the problems of large volume and being difficult to carry caused by including the cuff, the pump and the valve or the like structure can be avoid; the problems that the subject would feel discomfort because of the inflation and deflation of the cuff and the wrist tissues and blood vessels could be damaged because of the frequent compression from the cuff can also be avoided. The problem that the blood pressure continuous detection cannot be realized because the inflation and deflation of cuff need certain time can further be avoided. Compared with existing pulse wave detecting apparatuses, the problem that the positioning of the reference point may be inaccurate because the subject moves freely, and that the longtime and continuous detection cannot be realized can be also avoided.

Another purpose of the application is to provide a wearing apparatus. It aims to solve the problem in existing blood pressure detecting methods that there is a complicated structure, it is difficult to carry, a subject's skin may be damaged, the subject may be discomfort, the subject cannot move freely, the location is imprecise, and a continuous detection for a longtime cannot be realized.

In order to solve the above technical problems, the technical solution of the wearing apparatus provided by the application includes the above-described optical fiber continuous detecting blood pressure sensor.

The wearing apparatus provided by the application has the following advantages compared to the prior art:

Because the wearing apparatus has an optical fiber continuous detecting blood pressure sensor, when a person wears the wearing apparatus, the wrist or arm could move freely, and the 24 hours non-invasive continuous detection for the human blood pressure could be realized without repositioning.

### BRIEF DESCRIPTION OF THE DRAWINGS

Figure 1 is a cut-away view of an optical fiber continuous blood pressure sensor provided by one embodiment of the application;
Figure 2 is a perspective structural schematic view of the optical fiber continuous blood pressure sensor provided by the embodiment of the application;
Figure 3 is a layout of a first embodiment of the arrangement of the optical fiber of the optical fiber continuous detecting blood pressure sensor on the sensing band provided by the embodiment of the application;
Figure 4 is another layout of the first embodiment of the arrangement of the optical fiber of the optical fiber continuous detecting blood pressure sensor on the sensing band provided by the embodiment of the application;
Figure 5 is a layout of a fourth embodiment of the arrangement of the optical fiber of the optical fiber continuous detecting blood pressure sensor on the sensing band provided by the embodiment of the application;
Figure 6 is a layout of a second embodiment of the arrangement of the optical fiber of the optical fiber continuous detecting blood pressure sensor on the sensing band provided by the embodiment of the application;
Figure 7 is a layout of a third embodiment of the arrangement of the optical fiber of the optical fiber continuous detecting blood pressure sensor on the sensing band provided by the embodiment of the application;
Figure 8 is a rear view of the optical fiber continuous detecting blood pressure sensor which not includes a signal processor provided by the embodiment of the application;
Figure 9 is another rear view of the optical fiber continuous detecting blood pressure sensor which not includes the signal processor provided by the embodiment of the application;
Figure 10 is a layout of a fifth embodiment of the arrangement on the sensing band of the optical fiber of the optical fiber continuous detecting blood pressure sensor provided by the embodiment of the application.

### DETAILED DESCRIPTION OF THE PREFERRED EMBODIMENTS

In order to clearly describe the purpose, technical solutions and advantages of the present application, the application will be specifically described below with reference to the accompany drawings and embodiments. It should be understood that the embodiments described herein are merely specific embodiments for explaining the present application.

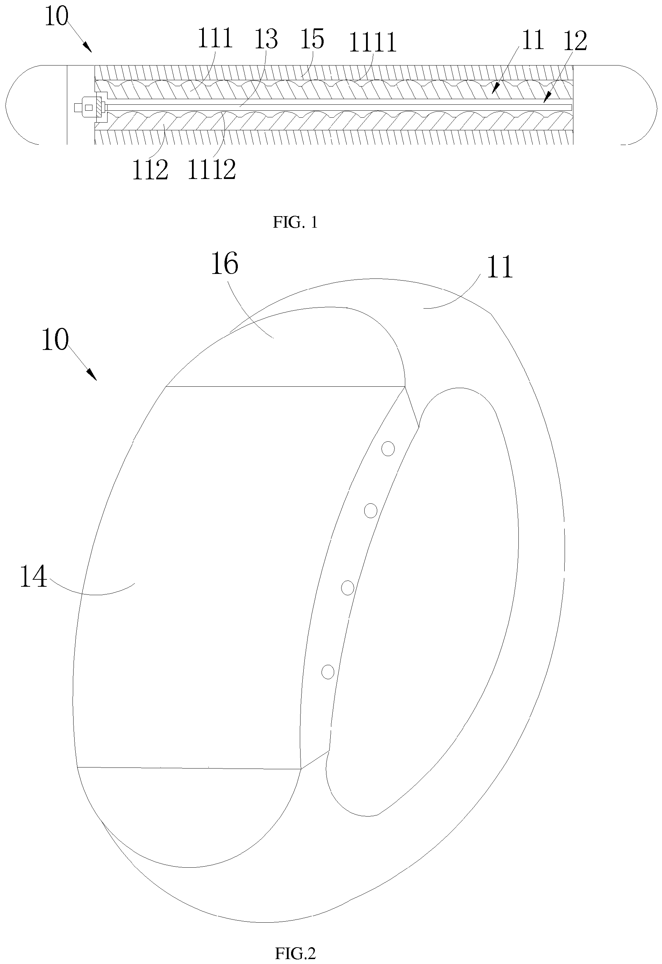

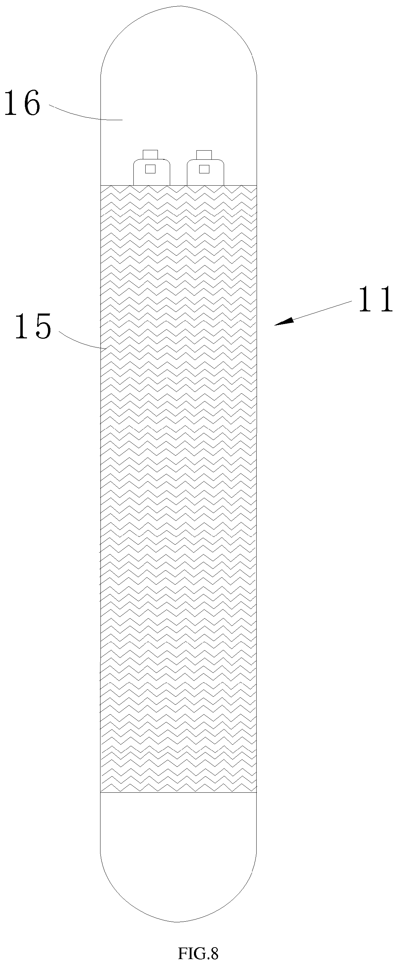

As shown in Figs.1-10, the preferred embodiments are provided by the application.

It should be noted that, when a part is referred to as "fixed" or "arranged" on another part, it could be on another part directly or there may be intermediate parts between the part and the another part; and when a part is referred to as "connected" with another part, it could be connected with another part directly or there may be intermediate parts between the part and the another part.

It should be noted that the position terms left, right, up and down or the like in the embodiment may merely be relative concepts or references to the normal working condition of the product, but not intended to limit the application.

As shown in Fig.1 and Fig.2, the optical fiber continuous detecting blood pressure sensor 10 provided by the application comprises a sensing band 11 which is flexible and can be curled into a ring. The sensing band 11 includes an inner layer 111 and an outer layer 112 that can be close to a human body. An accommodation space 12 is formed between the inner layer 111 and the outer layer 112. An optical fiber 13 is provided in the accommodation space 12 and arranged to extend along the sensing band 11 so as to form a sensing area to sense a pulse wave. A signal processor 14 is provided on one end of the sensing band and used to transform a light decaying signal generated by decaying a light signal through the optical 14 fiber into a charge unit so as to calculate the pulse wave. The signal processor 14 communicates with the optical fiber 13, and the outer surfaces of the inner layer and the outer layer are covered by an elastic layer. It should be noted that, as shown in Fig.1 and Fig.2, the reason why the sensing area could sense the pulse wave is that the fluctuation of the pulse of the artery would provide a fluctuant pressure on the sensing band 11. Correspondingly, the optical fiber 11 arranged in the accommodation space 12 of the sensing band 11 will deform due to the fluctuant pressure. Because of the deformation of the optical fiber 13, the transmission of the light signal in the optical fiber would generate larger refraction, reflection, scattering and the decaying variation. One end of the sensing band 11 is provided with a signal processor 14 which communicates with optical fiber 13. At the moment, the light decaying signal of the optical fiber 13 is transmitted to signal processor 14 through communication. Signal processor 14 transforms the light decaying signal into charge unit. Then the charge unit will be calculated into a pulse wave. Finally, the pulse wave is processed, analyzed and calculated to obtain the blood pressure value.

Compared with the prior art, the optical fiber continuous blood pressure sensor 10 provided by the present embodiment has the following advantages.

As shown in Fig.1 and Fig.2, the above said optical fiber continuous blood pressure sensor 10 is provided with a sensing band 11, the sensing band 11 has an inner layer and an outer layer. An accommodation space 12 is formed between the inner layer 111 and the outer layer 112 which is provided with optical fiber 13. Because the sensing band 11 is flexible and can be curled into a ring, an optical fiber can be arranged to extend along the sensing band, which will form a ring-shape sensing area. The sensing band could be worn on the human arm or wrist, especially worn on the area of human wrist radial artery. When the sensing band on human wrist rotates or dislocates because of the movement of a subject, the sensing area can sense the pulse wave of the human wrist radial artery, that is, the special positioning is not needed for wearing the sensing band.

Moreover, as shown in Fig.1 and Fig.2, the sensing band is covered by an elastic layer which can be contracted elastically at a certain extent. In this way, the sensing band can be worn on different human wrists. The elastic layer 15 has certain elasticity, which helps the sensing band 11 remain a certain elastic contact stress on the wrist. Therefore, the sensing band is worn on the wrist securely. During detecting the blood pressure, even the subject moves, the dislocation between the sensing band 11 and the human wrist radial artery would not be prone to occur and there is no need to reposition the sensing band 11, so that a 24 hours non-invasive continuous detection for a human blood pressure can be realized, which has high stability and precision.

Therefore, compared with existing non-invasive detecting methods, the problem that the volume is large and it is difficult to carry caused by including the cuff, the pump and the valve or the like structures can be avoid. The problems that the subject would feel discomfort because of the inflation and deflation of the cuff and the wrist tissues and blood vessels could be damaged because of the frequent compression from the cuff can also be avoided. The problem that the blood pressure continuous detection cannot be realized because the inflation and deflation of the cuff need certain time can be further avoided. Compared with existing pulse wave detecting apparatuses, the problem that the positioning of the reference point may be inaccurate because the subject moves freely, and that the longtime and continuous detection cannot be realized can also be avoided.

It should be noted that, the material of the sensing band 11 is soft material, such as silicone. The material of the elastic layer is elastic material, such as elastic woven material.

In this embodiment, a preferred embodiment about the specific structure of the outer surface of the inner layer 111 is shown in Fig.1 and Fig.2, in order to increase the elastic contact stress on the detected surface of the human wrist radial artery from the inner layer 111 of the sensing band 11, the outer surface of the inner layer 111 is formed with a first corrugated concave-convex structure 1111. In this way, when the sensing band 11 is worn on the human wrist, the first concave-convex structure 1111 of the outer surface of the inner layer 111 will reach the elastic layer 15, thereby reaching the detected surface. Under the elastic reaction of the elastic layer 15, the first concave-convex structure 1111 will be more closer to the detected surface, then the sensing band 11 would be more securely positioned on the wrist and not prone to cause the positioning variation because of the movement of the subject, thereby affecting the proceeding and precision of the continuous detection of the blood pressure.

As shown in Fig. 1 and Fig. 2, further, in order to increase the elastic contact stress on the detected surface of the human wrist radial artery from the inner layer 111 of the sensing band 11, the outer surface of the inner layer 112 is formed with a second corrugated concave-convex structure 1112. In this way, when the sensing band 11 is worn on the human wrist, the second concave-convex structure will reach the optical fiber 13 located in the accommodation space 12, and applies pressure towards the detected surface on the optical fiber. The pressure is transmitted by the inner layer 111 and the first concave-convex structure 1111, finally acts on the detected surface through the elastic layer 15 and makes the elastic layer 15 more closer to the detected surface, then the sensing band 11 would be more securely positioned on the wrist, and it is ensured that the positioning of the sensing band 11 would not be prone to be affected by the movement of the subject, so that the proceeding and precision of the continuous detecting of the blood pressure will be guaranteed.

Specifically, as shown in Fig. 1, the shape of the first convex-concave structure is selected from a triangular corrugated shape, a circular corrugated shape, a quadrate corrugated shape, a trapezoidal corrugated shape, and any combination of at least two of these shapes. In like manner, the shape of the second concave-convex structure 1112 is similar to the shape of the first concave-convex structure 1111. The shapes of the second concave-convex structure 1112 and the first concave-convex structure 1111 only need to meet the requirement of being able to abut against the detected surface.

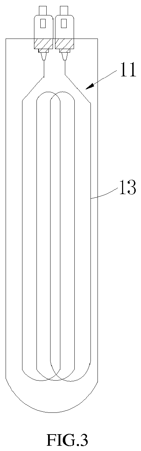

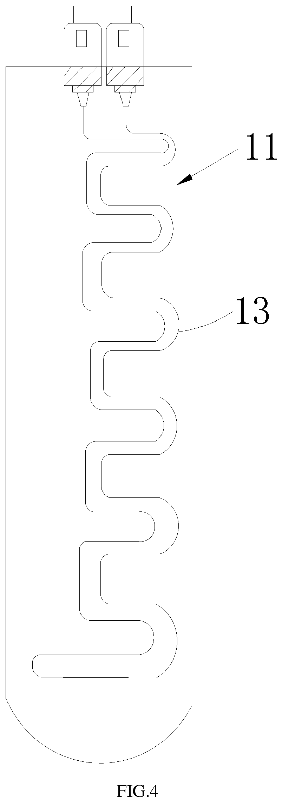

Specifically, the first embodiment about the specific arrangement of the optical fiber which is located in the accommodation space, as shown in Fig.3 and Fig.4, the optical fiber 13 includes an optical fiber which has many U-shape concatenated cablings extending along the longitudinal direction or width direction of the sensing band 11.

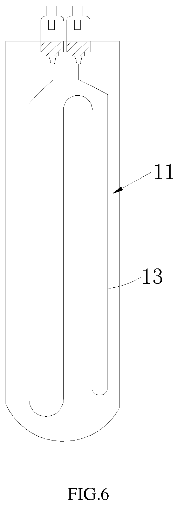

A second embodiment about the specific arrangement of the optical fiber located in the accommodation space, as shown in Fig.6, the optical fiber 13 includes an optical fiber which has many S-shape concatenated cablings extending along the longitudinal direction or width direction of the sensing band 11.

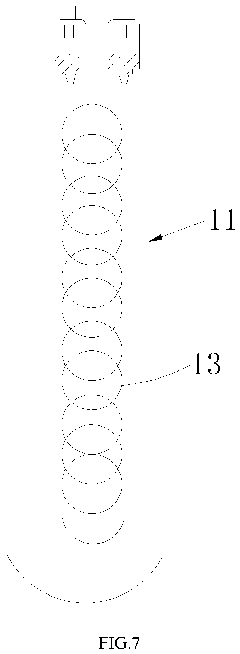

A third embodiment about the specific arrangement of the optical fiber located in the accommodation space, as shown in Fig.7, the optical fiber 13 includes an optical fiber which has many O-shape concatenated cablings extending along the longitudinal direction or width direction of the sensing band 11.

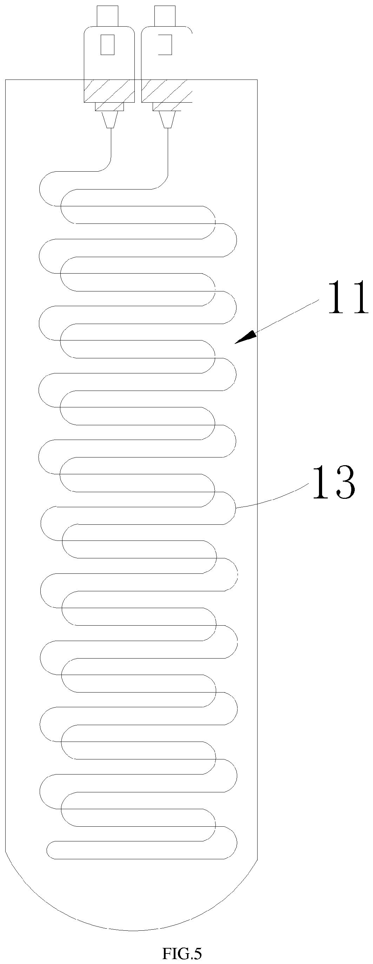

A fourth embodiment about the specific arrangement of the optical fiber located in the accommodation space, as shown in Fig.5, at least two optical fiber rows are superposed vertically, and bending portions of the cablings of the two vertically superposed and adjacent optical fiber rows are staggered.

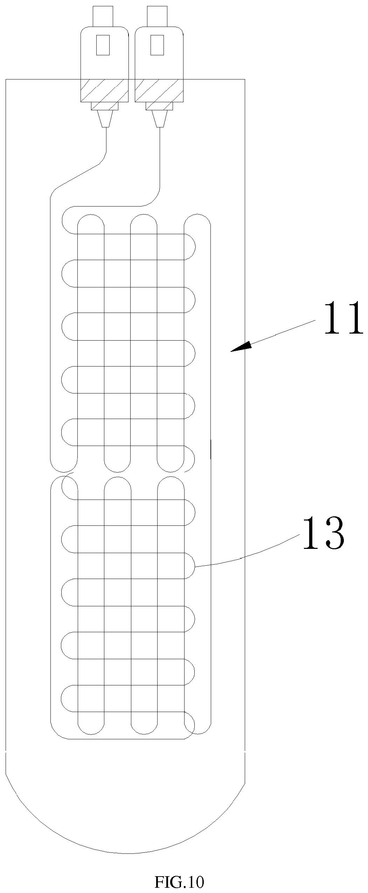

The fifth embodiment about the specific arrangement of the optical fiber located in the accommodation space, as shown in Fig.10, at least two optical fiber rows are superposed vertically, and bending portions of the cablings of the two vertically superposed and adjacent optical fiber rows are staggered transversely and longitudinally.

Whichever one of the above five embodiments is adopted by the arrangement of the optical fiber 13, the ribbon sensing area of the optical fiber 13 extending along the sensing band 11 is intensive and uniform. In this way, it helps increase the sensing sensitivity of the sensing area to the pulse wave of the human wrist radial artery . No matter what the contact stress from the inner layer 111 of the sensing band 11 to the detected surface is inadequate or decreases, the sensing area could sense the pulse wave of the radial artery sensitively and helps improve the precision of blood pressure detecting.

The preferred embodiment about the specific structure of the signal processor 14. Further, the signal processor includes an optical detecting module (not shown) used to receive the light decay signal of the optical fiber; a signal calculating and processing module (not shown) used to transform the light decaying signal into the charge unit so as to calculate the pulse wave; a blood pressure calibrating module (not shown) used to process, analyze and calculate the pulse wave so as to obtain the blood pressure value; a memory module used to store the blood pressure value; and a displaying module (not shown) used to display the blood pressure value.

It should be noted that, the displaying module can display by a fixed terminal or a mobile terminal. For example, the display module can be a computer screen, a laptop screen, a cellphone screen or an Ipad screen, etc.

Specifically, the signal processor 14 includes a communicating module (not shown) which transmits the blood pressure value signal and other signals to the fixed terminal or mobile terminal through wire communication or wireless communication.

Specifically, wire communication can communicate with the terminal through ports and wireless communication can communicate with the terminal through a bluetooth module. A fixed terminal or a mobile terminal, such as a computer or a cellphone can record, stores the detected blood pressure value or other values, thereby forming a longterm continuous recording and creating a searchable report. Moreover, the report containing blood pressure value or other values can be stired to the cloud, then the report can be downloaded from the cloud as medical diagnostic reference.

As shown in Fig.1 and Fig.2 , the two ends of the inner layer 111 and the outer layer 112 are connected to the two ends of the signal processor respectively. The outer surface of the inner layer 112 is provided with a fixed structure 16 which connects the outer layer 112 and the signal processor 14 fixedly. The material of the fixed structure 16 can be bonding material, such as velcro etc.

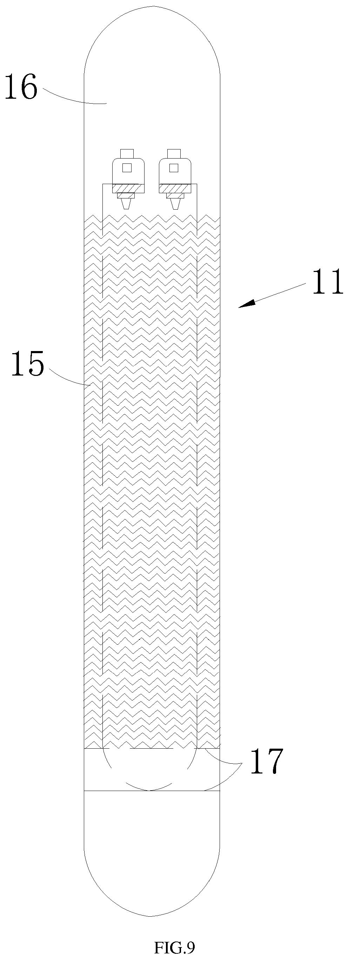

As shown in Fig.1 and Fig. 9, the inner surface of the inner layer 111 or the inner surface 112 of the outer surface is provided with a internal pocket 17. One end of the ribbon sensing area formed by the optical fiber which extends along the sensing area 11 is inserted into the internal pocket 17. In this way, the optical fiber 13 can be arranged in the accommodation space 12 securely.

The signal processor 14 is provided with an alarming module (not shown). The alarming module can be a siren or the like. In this way, the blood pressure value obtained from the blood pressure calibrating module can be transmitted to the alarming module. If the blood pressure value exceeds a normal range preset in the alarming module, the alarm will be triggered and give voice alarm. The normal range of the blood pressure value can be personalized and set individually. Therefore, the alarming module has automatic identification function.

The application of the above optical fiber continuous detecting blood pressure sensor 10: it can be manufactured as a waterproof film wrist. The shape and size of the waterproof film can be adjusted according to the wrists of different people or the different locations of the detected radial arteries. The connector of the optical fiber can be designed as thin and small types, so that the optical fiber continuous detecting blood sensor can be further miniaturized and easy to wear. Besides detecting the blood pressure, the above optical fiber continuous detecting blood sensor can be used for detecting heart rate, breathing, sleep, calorie consuming, etc.

The embodiment further provides a wearing apparatus (not shown), which includes an optical fiber continuous detecting blood pressure sensor 10.

Compared with the prior art, the wearing apparatus provided by the embodiment has the following advantages:
Because the wearing apparatus has an optical fiber continuous detecting blood pressure sensor 10, when a person wears the wearing apparatus, the wrist or arm could move freely, and 24 hours non-invasive continuous detection for the human blood pressure could be realized without repositioning.

## Claims

1. An optical fiber continuous detecting blood pressure sensor (10), which comprises: a sensing band (11) which is flexible and can be curled into a ring, the sensing band (11) including an inner layer (111) which abuts against the detected surface and an outer layer (112); an accommodation space (12) formed between the inner layer (111) and the outer layer (112); an optical fiber (13) provided in the accommodation space (12) and extending along the sensing band (11) so as to form a sensing area to sense a pulse wave; a signal processor (14) provided on one end of the sensing band (11) and configured to convert a luminous decay signal generated by passing a light signal through the optical fiber (13) to be decayed into a charge unit so as to calculate the pulse wave; the signal processor (14) communicated with the optical fiber, and an outer surface of the sensing band being covered by an elastic layer (15); **characterized in that**, a corrugated first concave-convex structure (1111) is formed on an outer surface of the inner layer (111).

2. The optical fiber continuous detecting blood pressure sensor (10) according to claim 1, **characterized in that**, the shape of the first convex-concave structure (1111) is selected from a group consisting of a triangular corrugated shape, a circular corrugated shape , a quadrate corrugated shape, and a trapezoidal corrugated shape, or from any combination of the triangular corrugated shape, the circular corrugated shape, the quadrate corrugated shape and the trapezoidal corrugated shape.

3. The optical fiber continuous detecting blood pressure sensor (10) according to claim 2, **characterized in that**, the optical fiber (13) includes an optical fiber row which has several U-shape concatenated cabling extends along the longitudinal direction or width direction of the sensing band (11).

4. The optical fiber continuous detecting blood pressure sensor (10) according to claim 2, **characterized in that**, the optical fiber (13) includes an optical fiber row which has several S-shape concatenated cablings extending along the longitudinal direction or width direction of the sensing band (11).

5. The optical fiber continuous detecting blood pressure sensor (10) according to claim 2, **characterized in that**, the optical fiber (13) includes an optical fiber row which has several O-shape concatenated cablings extending along the longitudinal direction or width direction of the sensing band (11).

6. The optical fiber continuous detecting blood pressure sensor (10) according to any one of claim 3-5, **characterized in that**, the signal processor (14) includes an optical detecting module configured to receive the light decay signal of the optical fiber (13), a signal calculating and processing module configured to convert the light decay signal of the optical fiber (13) into charge unit so as to calculate the pulse wave, a blood pressure calibrating module configured to process, analyze and calculate the pulse wave in order to obtain the blood pressure value, a memory module configured to store the blood pressure value, and a displaying module configured to display the blood pressure value.

7. The optical fiber continuous detecting blood pressure sensor (10) according to claim 6, **characterized in that**, at least two optical fiber rows are superposed vertically, and bending portions of the cablings of the two vertically superposed and adjacent optical fiber rows are staggered.

8. The optical fiber continuous detecting blood pressure sensor (10) according to claim 6, **characterized in that**, at least two optical fiber rows are superposed vertically, and bending portions of the cablings of the two vertically superposed and adjacent optical fiber rows are staggered transversely and longitudinally.

9. A wearing apparatus, **characterized in that**, the wearing apparatus comprises the optical fiber continuous detecting blood pressure sensor (10) according to any one of claims 1-8.

## Patentansprüche

1. Faseroptischer kontinuierlicher Blutdruckdetektionssensor (10), welcher umfasst:
ein Abfühlband (11), das flexibel ist und zu einem Ring eingerollt werden kann, wobei das Abfühlband (11) einschließt: eine innere Schicht (111), die an der detektierten Oberfläche anliegt, und eine äußere Schicht (112); einen Unterbringungsraum (12), der zwischen der inneren Schicht (111) und der äußeren Schicht (112) gebildet ist; eine optische Faser (13), die in dem Unterbringungsraum (12) bereitgestellt wird und sich entlang des Abfühlbands (11) erstreckt, um so einen Abfühlbereich zu bilden, um eine Pulswelle abzufühlen;
einen Signalprozessor (14), der an einem Ende des Abfühlbands (11) bereitgestellt wird und konfiguriert ist, um ein Lichtabschwächungssignal, das generiert wird, indem ein Lichtsignal durch die optische Faser (13) geleitet wird, wo es sich abschwächt, in eine Ladungseinheit zu konvertieren, um so die Pulswelle zu berechnen; wobei der Signalprozessor (14) mit der optischen Faser kommuniziert und eine Außenoberfläche des Abfühlbands von einer Elastikschicht (15) bedeckt ist;
**dadurch gekennzeichnet, dass** auf einer Außenoberfläche der inneren Schicht (111) eine geriffelte erste konkav-konvexe Struktur (1111) gebildet ist.

2. Faseroptischer kontinuierlicher Blutdruckdetektionssensor (10) nach Anspruch 1, **dadurch gekennzeichnet, dass** die Form der ersten konvex-konkaven Struktur (1111) ausgewählt ist aus einer Gruppe bestehend aus einer dreieckigen geriffelten Form, einer kreisförmigen geriffelten Form, einer quadratischen geriffelten Form und einer trapezförmigen geriffelten Form, oder aus jedweder Kombination der dreieckigen geriffelten Form, der kreisförmigen geriffelten Form, der quadratischen geriffelten Form und der trapezförmigen geriffelten Form.

3. Faseroptischer kontinuierlicher Blutdruckdetektionssensor (10) nach Anspruch 2, **dadurch gekennzeichnet, dass** die optische Faser (13) eine optische Faserreihe einschließt, die etliche U-förmige verkettete Verkabelungen aufweist, die sich entlang der Längsrichtung oder Breitenrichtung des Abfühlbands (11) erstrecken.

4. Faseroptischer kontinuierlicher Blutdruckdetektionssensor (10) nach Anspruch 2, **dadurch gekennzeichnet, dass** die optische Faser (13) eine optische Faserreihe einschließt, die etliche S-förmige verkettete Verkabelungen aufweist, die sich entlang der Längsrichtung oder Breitenrichtung des Abfühlbands (11) erstrecken.

5. Faseroptischer kontinuierlicher Blutdruckdetektionssensor (10) nach Anspruch 2, **dadurch gekennzeichnet, dass** die optische Faser (13) eine optische Faserreihe einschließt, die etliche O-förmige verkettete Verkabelungen aufweist, die sich entlang der Längsrichtung oder Breitenrichtung des Abfühlbands (11) erstrecken.

6. Faseroptischer kontinuierlicher Blutdruckdetektionssensor (10) nach einem der Ansprüche 3 bis 5, **dadurch gekennzeichnet, dass** der Signalprozessor (14) ein optisches Detektionsmodul, das konfiguriert ist, um das Lichtabschwächungssignal der optischen Faser (13) zu empfangen, ein Signalberechnungs- und -verarbeitungsmodul, das konfiguriert ist, um das Lichtabschwächungssignal der optischen Faser (13) in eine Ladungseinheit zu konvertieren, um so eine Pulswelle zu berechnen, ein Blutdrucckalibrierungsmodul, das konfiguriert ist, um die Pulswelle zu verarbeiten, zu analysieren und zu berechnen, um den Blutdruckwert zu erhalten, ein Speichermodul, das konfiguriert ist, um den Blutdruckwert zu speichern, und ein Anzeigemodul einschließt, das konfiguriert ist, um den Blutdruckwert anzuzeigen.

7. Faseroptischer kontinuierlicher Blutdruckdetektionssensor (10) nach Anspruch 6, **dadurch gekennzeichnet, dass** mindestens zwei optische Faserreihen vertikal übereinander gelegt sind und Biegeabschnitte der Verkabelungen der beiden vertikal übereinander gelegten und benachbarten optischen Faserreihen gestaffelt sind.

8. Faseroptischer kontinuierlicher Blutdruckdetektionssensor (10) nach Anspruch 6, **dadurch gekennzeichnet, dass** mindestens zwei optische Faserreihen vertikal übereinander gelegt sind und Biegeabschnitte der Verkabelungen der beiden vertikal übereinander gelegten und benachbarten optischen Faserreihen in Querrichtung und in Längsrichtung gestaffelt sind.

9. Tragegerät, **dadurch gekennzeichnet, dass** das Tragegerät den faseroptischen kontinuierlichen Blutdruckdetektionssensor (10) nach einem der Ansprüche 1 bis 8 umfasst.

## Revendications

1. Capteur de tension artérielle à détection continue par fibre optique (10), qui comprend :
une bande de détection (11) qui est souple et peut être enroulée en un anneau, la bande de détection (11) comportant une couche interne (111) qui s'appuie contre la surface détectée et une couche externe (112) ; un espace d'accueil (12) formé entre la couche interne (111) et la couche externe (112) ; une fibre optique (13) disposée dans l'espace d'accueil (12) et s'étendant le long de la bande de détection (11) de manière à former une zone de détection pour détecter une onde de pouls ; un processeur du signal (14) disposé sur une extrémité de la bande de détection (11) et configuré pour convertir un signal de décroissance lumineuse généré par passage à travers la fibre optique (13) d'un signal lumineux à faire décroître en une unité de charge de manière à calculer l'onde de pouls ; le processeur du signal (14) étant en communication avec la fibre optique, et une surface externe de la bande de détection étant recouverte par une couche élastique (15) ;
**caractérisé en ce qu'**une première structure ondulée concave-convexe (1111) est formée sur une surface externe de la couche interne (111).

2. Capteur de tension artérielle à détection continue par fibre optique (10) selon la revendication 1, **caractérisé en ce que** la forme de la première structure ondulée concave-convexe (1111) est choisi dans un groupe constitué par une forme ondulée triangulaire, une forme ondulée circulaire, une forme ondulée quadrangulaire et une forme ondulée trapézoïdale, ou parmi toutes les combinaisons de la forme ondulée triangulaire, la forme ondulée circulaire, la forme ondulée quadrangulaire et la forme ondulée trapézoïdale.

3. Capteur de tension artérielle à détection continue par fibre optique (10) selon la revendication 2, **caractérisé en ce que** la fibre optique (13) comporte une ligne de fibre optique qui a plusieurs câbles en forme de U concaténés s'étendant le long de la direction longitudinale ou la direction de la largeur de la bande de détection (11).

4. Capteur de tension artérielle à détection continue par fibre optique (10) selon la revendication 2, **caractérisé en ce que** la fibre optique (13) comporte une ligne de fibre optique qui a plusieurs câbles en forme de S concaténés s'étendant le long de la direction longitudinale ou la direction de la largeur de la bande de détection (11).

5. Capteur de tension artérielle à détection continue par fibre optique (10) selon la revendication 2, **caractérisé en ce que** la fibre optique (13) comporte une ligne de fibre optique qui a plusieurs câbles en forme de O concaténés s'étendant le long de la direction longitudinale ou la direction de la largeur de la bande de détection (11).

6. Capteur de tension artérielle à détection continue par fibre optique (10) selon l'une quelconque des revendications 3 à 5, **caractérisé en ce que** le processeur du signal (14) comporte un module de détection optique configuré pour recevoir le signal de décroissance lumineuse de la fibre optique (13), un module de calcul et traitement du signal configuré pour convertir le signal de décroissance lumineuse de la fibre optique (13) en unité de charge de manière à calculer l'onde de pouls, un module d'étalonnage de tension artérielle configuré pour traiter, analyser et calculer l'onde de pouls afin d'obtenir la valeur de tension artérielle, un module de mémoire configuré pour stocker la valeur de tension artérielle, et un module d'affichage configuré pour afficher la valeur de tension artérielle.

7. Capteur de tension artérielle à détection continue par fibre optique (10) selon la revendication 6, **caractérisé en ce qu'**au moins deux lignes de fibre optique sont superposées verticalement, et des parties courbes des câbles des deux lignes de fibre optique superposées verticalement et adjacentes sont disposées en quinconce.

8. Capteur de tension artérielle à détection continue par fibre optique (10) selon la revendication 6, **caractérisé en ce qu'**au moins deux lignes de fibre optique sont superposées verticalement, et des parties courbes des câbles des deux lignes de fibre optique superposées verticalement et adjacentes sont disposées en quinconce transversalement et longitudinalement.

9. Appareil à porter, **caractérisé en ce que** l'appareil à porter comprend le capteur de tension artérielle à détection continue par fibre optique (10) selon l'une quelconque des revendications 1 à 8.
